(19) Europäisches Patentamt / European Patent Office / Office européen des brevets

(11) **EP 4 427 746 A1**

(12) **EUROPEAN PATENT APPLICATION**

(43) Date of publication:
**11.09.2024 Bulletin 2024/37**

(21) Application number: **23160766.4**

(22) Date of filing: **08.03.2023**

(51) International Patent Classification (IPC):
**A61K 31/4184** (2006.01)     **A61K 31/69** (2006.01)
**A61K 38/00** (2006.01)     **A61P 25/28** (2006.01)

(52) Cooperative Patent Classification (CPC):
**A61K 31/69; A61K 31/4184; A61P 25/28**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Bash Biotech Inc**
**San Diego, CA 92101 (US)**

(72) Inventors:
• **MARDINOGLU, Adil**
**412 60 Göteborg (SE)**
• **LI, Xiangyu**
**113 46 Stockholm (SE)**

(74) Representative: **Kransell & Wennborg KB**
**P.O. Box 27834**
**115 93 Stockholm (SE)**

(54) **TREATMENT OF DEMENTIA USING BORTEZOMIB OR PARBENDAZOLE**

(57)     There is provided a product for use in a method of prevention or treatment of dementia, wherein the product is or bortezomib or parbendazole.

Fig. 6

## Description

### TECHNICAL FIELD

The present disclosure relates to the field of treatment of dementia, in particular Alzheimer's disease (AD).

### BACKGROUND

After numerous clinical trials and decades of endeavour, there is still no effective cure for AD or any other type of dementia. These diseases are among the most prevalent public health threats due to incrementally ageing populations.

Bortezomib is a chemotherapy drug used to treat multiple myeloma, a cancer that affects the plasma cells in the bone marrow, as well as mantle cell lymphoma, a type of non-Hodgkin lymphoma. The drug works by blocking the action of an enzyme called proteasome, which is necessary for the growth and survival of cancer cells. Bortezomib belongs to a class of drugs called proteasome inhibitors, which target the proteasome pathway in the cancer cells, preventing them from growing and dividing.

Bortezomib is available as an injection and administered over four to five minutes by a doctor or health care professional.

Parbendazole is a broad-spectrum anthelmintic compound used to treat a variety of parasitic infections in humans, livestock, and poultry. This drug works by inhibiting the production of microtubule proteins that are necessary for the survival of nematodes and cestodes. It is effective against nematodes and cestodes, with a wide range of activity against gastrointestinal worms (ascarids, hookworms and whipworms), tapeworms, flukes, and trematodes.

Parbendazole has been found to be safe and well-tolerated in humans, livestock and poultry. The drug is generally well-absorbed following oral administration and displays a dose-dependent bioavailability. The elimination of parbendazole is mainly renal and is excreted in both urine and faeces. It displays an antagonistic action against the enzymes that are responsible for the survival and reproduction of the parasitic organisms.

### SUMMARY

Exploring the novel use of the previously approved and investigational molecules by using computational drug repurposing approach together with systems medicine approach provide a promising solution to the problems of the prior art. In general, the pharmacokinetics, bioavailability and possible side effects of repurposed drugs have been well-characterised, which simplifies the regulatory procedures for approved drugs, and reduces the cost and duration compared to *de novo* drug development (Ko, 2020). For instance, a series of drugs that have been previously approved for the treatment of hypertension, diabetes, epilepsy, nausea and several other diseases have undergone clinical trials for the treatment of AD (Hascup et al., 2021; Jourdan et al., 2020; Matthews et al., 2021; Vossel et al., 2021). These drugs have attenuated AD symptoms to remarkable degrees in early phases by targeting numerous clinical parameters other than tau or amyloid-beta. This endorses the conceptual shift in the amyloid-centred definition of AD and the transition to the drug repositioning in the field (Mullane and Williams, 2020).

Drug repurposing usually benefits an integrated analysis of large-scale biological, biomedical, and electronic health-related data by the use of high computation capabilities and state-of-the-art algorithms. The richness of data resources has resulted in various drug repurposing strategies. In general, drug repurposing strategies can be classified by how data is utilised (data-based, network-based or profile-based). Profile-based drug repurposing strategy supposes that pharmacologic perturbations caused by small molecules are comparable even under varying biological conditions since repurposed small molecules share similar therapeutic mechanisms or modes of action (MOAs) (Ko, 2020). Furthermore, no prior knowledge of drugs or diseases is required to identify these mechanisms, making the profile-based approach applicable to new compounds. The massive increase in gene expression profiles and resources allows for an increase in profile-based drug repositioning studies (Cheng et al., 2019; Clough and Barrett, 2016; Culhane et al., 2012; Subramanian et al., 2017; Wu et al., 2006).

The Connectivity Map (CMap) is an extensive example of a resource for high-throughput profiles (Subramanian et al., 2017). The database collects gene expression signature profiles (i.e., perturbation signature profiles or perturbagens) of different human cell lines perturbed by thousands of compounds, gene overexpression or inhibition. The mapping of drug-perturbed perturbation and disease-induced perturbation on human cells is a commonly used profile-based method to discover potentially effective drugs against the diseases. Correlation and anti-correlation of perturbation profiles are effective measurements in this mapping (Glicksberg et al., 2019; Ko, 2020). This approach has been successful in identifying potential drugs to use against diseases such as clear cell renal carcinoma, liver cancer, SARS-COV-2, and AD (Bonnet et al., 2022; Li et al., 2022a; Nevado-Holgado and Lovestone, 2017; Yuan et al., 2022).

In the study behind the present disclosure, the inventors applied their drug repositioning method in AD and evaluated the similarity of the perturbations induced by the target gene knockdown and candidate drug treatment on the available human cell lines. Reference is made to a preliminary systems biology study, where genetic and metabolic changes occurring in the AD brain were examined using gene co-expression network (GCN) analysis and genome-scale metabolic modelling (Bayraktar et al., 2021). To the inventors' knowledge, drug repositioning studies using perturbagens are targeted to a limited set of drugs on certain cell lines, where the condition occurs; this brings two handicaps: i) drug repositioning is underperforming due to being restricted on under-studied cell lines, ii) fewer compounds are screened and restrains the potential solutions for many conditions including the AD (Chen et al., 2013; Rangaraju et al., 2018; Williams et al., 2019). The use of multiple cell lines is a good practice preferred in cancer cell lines thanks to the similar drug MOAs on multiple diseases (Lee et al., 2016). Hence, the drug repositioning approach has been applied based on the transcriptomics data from multiple non-glial/non-neural tissues. Further computational tests have been performed to investigate the target gene druggability, candidate drug bioavailability, functional enrichment of drug-perturbed profiles and target gene-drug associations. Lastly, *in vitro* experiments have been conducted to test and validate the efficacy and toxicity of two of the drugs, bortezomib and parbendazole.

Accordingly, the present disclosure provides bortezomib or parbendazole for use in a method of prevention or treatment of dementia, such as Alzheimer's disease (AD).

## BRIEF DESCRIPTION OF THE FIGURES

Fig. 1 is a heatmap showing the log-2 fold changes of *GLS, KLC1* and *NDRG1*, which have been overexpressed either on the dorsolateral prefrontal cortex, temporal cortex or cerebellum. The colour transition indicates overexpression in AD samples. Significantly expressed genes (adjusted p-value < 0.05) labelled with "*".

Fig. 2 is a boxplot for *GLS* essentiality scores from Chronos dataset on multiple tissues from nervous system cell lineage or representing seven studied cell lineages. Boxes represent the interquartile ranges where a median is a middle vertical line in a box. An essential score closer to -0.5 represents knockdown-dependent depletion and a score closer to -1 represents knockdown-dependent obliteration, whereas a zero score implies non-essentiality and a positive score may indicate cell proliferation or may appear as a random annotation.

Fig. 3 shows correlation coefficients for drug candidates identified as described in the Examples section below.

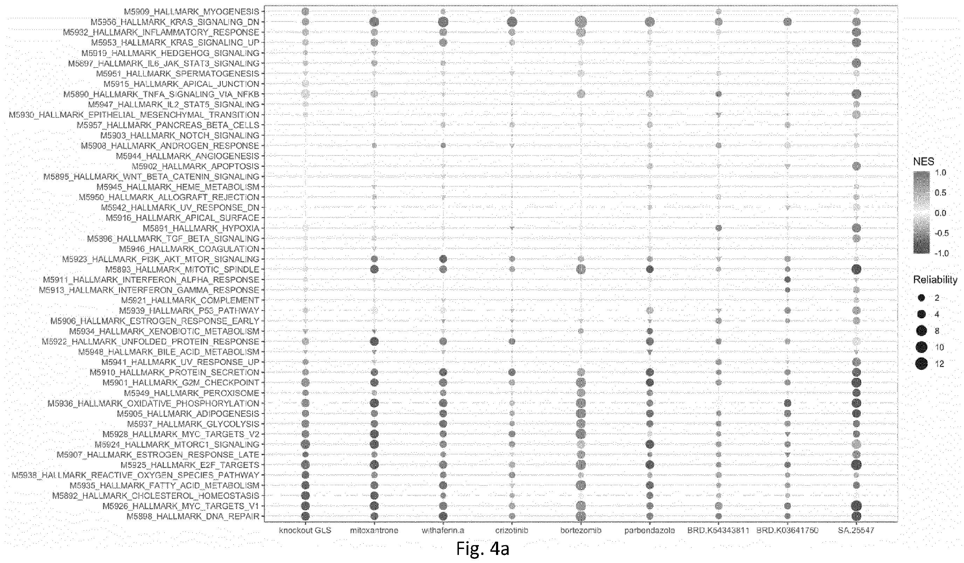

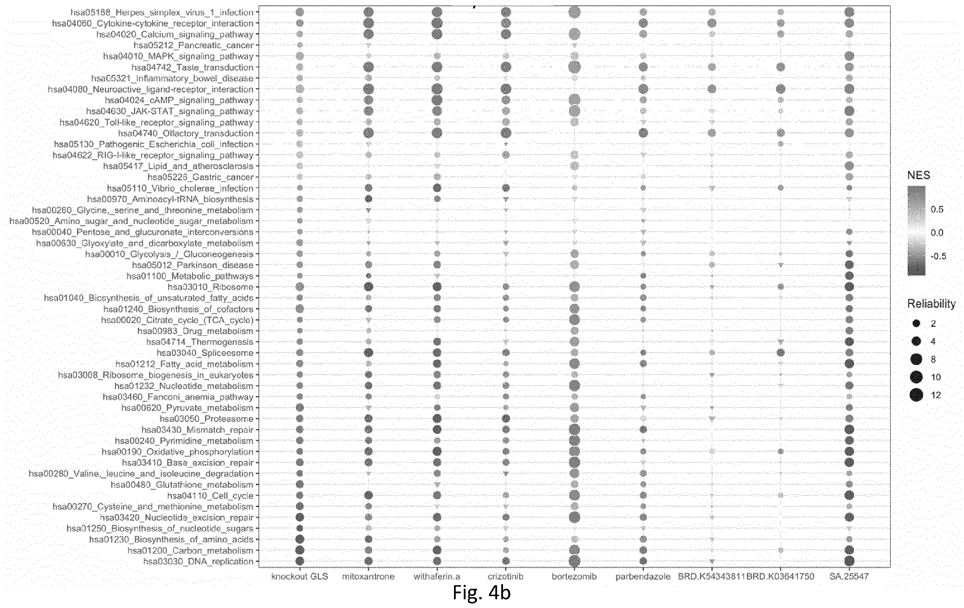

Fig. 4 shows bubble plots for aggregated pathway enrichments induced by GLS knockdown and candidate drugs, considering (up) MSigDB hallmark pathways (n=50) and (down) KEGG pathways significantly changed by GLS knockdown (n=61). In the upper part of the figure, the colour shows a larger normalised enrichment score (NES) / enrichment. In the lower part of the figure, the colour shows a larger negative NES / repression. The size of the "bubble" represents higher reliability, which is -log(FDR adjusted and weighted Fisher aggregated p-value) for enrichments. The circle represents significant enrichments/repressions, and the triangle represents non-significant enrichment/repressions.

Fig. 5 shows enrichment patterns for randomly given drugs.

Fig. 6 shows metabolic activity and cytotoxicity results obtained by MTT assay and LDH release assay.

Fig. 7 shows glutamate content compared to cell viability.

Fig. 8 is a western blot showing the intensities for glutaminase (GLS and GLS2) and GAPDH (negative control) levels by drug introduction.

## DETAILED DESCRIPTION

*GLS* has been identified as a key gene for the treatment of AD based on GCN analysis, reporter metabolite analysis and essentiality analysis in different tissues. A profile-based method based on the correlation of gene knockdown or drug-induced transcription profiling of cell lines for AD treatment by suppressing the *GLS*-dependent synthesis of glutamate and glutamate signalling is presented. A functional and physical link between candidate drugs and *GLS* has been found by applying an aggregated GSE analysis, investigating customly united interactome of DPIs/PPIs and performing *in vitro* experiments

The conversion of glutamine to glutamate and other amino acids (i.e., glutaminolysis) is quite critical in ageing tissues. On the one hand, glutaminolysis occurs as a pH-balancing mechanism in the senescent cells and improve the survival (Johmura et al., 2021). On the other hand, the same mechanism may reduce the glutamate reuptake capacity of astrocytes, by inducing senescence. Further, this induces post-synaptic NMDARs, leading to potassium influx in neurons, and irreversibly disrupts the ion balance (Sikora et al., 2021). Therefore, regulating glutaminases appears as an important checkpoint for elder health besides AD.

Glutaminase isoforms have been targeted in the treatment of different cancers. The study behind the present disclosure also highlighted the common goals in cancer research and ageing associated metabolic disorders by indicating glutaminase as a critical target in AD patients. Herein, drug candidates that target glutaminase isoforms via cell cycle regulatory elements and rebalance glutamateinduced disruption in ion homeostasis are discovered.

Eight candidate drugs were identified and further examined. Three of these compounds (BRD-K5433811, BRD-K03641750, SA-25547) have been understudied. Nevertheless, the study put forth their promising therapeutic effects; SA-25547 and BRD-K5433811 were predicted to be available to the brain, and the former had functional enrichments fitting glutaminase suppression. Still, they need to be tested experimentally further to elaborate their biological use. The other five drugs are well-studied anti-cancer drugs (bortezomib, crizotinib, mitoxantrone, withaferin-a) and an anthelmintic drug (parbendazole) whose mechanisms and adverse effects are known. Computational analyses indicated their ability as regulators of glutaminase and glutamate levels. The effect of bortezomib and parbendazole has been shown based on *in vitro* experiments.

Cancer and neurodegeneration are often associated inversely, both sharing similarities on the opposite ends of biological events. But accumulating pieces of evidence has caused a paradigm shift; many hallmarks of cancer can be common to neurodegeneration, at the onset and during the progression of neurodegeneration, specifically for AD. Reduced AD risk in patients taking anti-cancer drug treatment and ongoing clinical trials on neurodegenerative disorders assist this shift in the cancer-neurodegeneration axis (Ancidoni et al., 2021; Wrasidlo et al., 2014). Important cancer markers (e.g. p53, HIF1 and SIRT7) are demonstrated to induce glutaminase upregulation (Choudhury et al., 2020; Hu et al., 2010; Xiang et al., 2019). In addition, glutamate is often depicted as a major component of cancer metabolic reprogramming, considering the wide and crucial roles of glutamine and glutamate in energy production, amino acid biosynthesis and signal regulations; hence its isoforms have been targeted for the treatment of numerous cancer (Matés et al., 2019). Although drug-target interactome could not show the full extent of transcription factors and signalling proteins affecting GLS, enrichment of many cancer signalling pathways (e.g., Ras, MAPK and JAK-STAT) underlines this strong interplay between GLS and these proteins, and hereby associated with mitochondrial metabolism, immune response and cell proliferation.

Indeed, there is evidence for the effect of these anti-cancer drugs on CNS. For instance, mitoxantrone has shown positive effect on neurodegeneration in a longitudinal study of a multiple sclerosis cohort (Chartier et al., 2018). It has also been reported that Withaferin-a activates the transcription of several cytoprotective enzymes, such as SOD1, CAT and GPX, and reduces the level of pro-inflammatory metabolites by inhibiting NF-kB activity that further attenuates TNF-a and COX-2 in rat brains (Lee and Choi, 2016).

Some other evidence is less clear for other drug candidates but still revolves around common cancer/AD hallmarks. Crizotinib inhibits MET tyrosine kinase receptor (Shaw et al., 2011), whose native ligand, hepatocyte growth factor (HGF), induces extracellular signal-regulated kinase (ERK), and glutaminase indirectly. HGF/MET activation does not only effect glutaminases but also effect the various downstream proliferative signalling pathways. Moreover, HGF boosts glutamate receptor expression, synaptic plasticity and the transmission in the hippocampal neurons (Sharma, 2010). Bortezomib is a proteasomal inhibitor and potentially an important modulator of autophagy (Eshraghi et al., 2022), which is postulated to be vital for healthy ageing and treatment of AD (Aunan et al., 2017). Bortezomib also inhibits RELA (NF-kB subunit) and hence inactivates glutaminase.

An anti-parasitic drug candidate parbendazole shows similar controversial properties, but has a major advantage; it can penetrate BBB easily. Parbendazole degenerates microtubules of nematodes by targeting tubulin monomers as given in the interactome screening (Al-Hadiya, 2010). Conversely, immunocytochemistry screening of parbendazole and other anthelmintic drugs have shown increased myelin repair and oligodendroglial cell differentiation in rat and human cell cultures (Manousi et al., 2021). In addition to this proliferative effect, parbendazole may inhibit monoamine oxidase (MAO) and interfere with energy production (Al-Hadiya, 2010). MAO inhibitors inactivate monoamine neurotransmitters (e.g., dopamine, serotonin, melatonin) and they are used in psychiatric diseases, AD and PD (Dezsi and Vecsei, 2017; Finberg and Rabey, 2016). More interestingly, glutamate is often co-transmitted from all types of neurons together with primary neurotransmitters (Trudeau and El Mestikawy, 2018); therefore, the use of MAO inhibitors or parbendazole may also regulate the imbalance in glutamate levels.

Accordingly, as a first aspect of the present disclosure, there is provided a product for use in a method of prevention or treatment of dementia, wherein the product is or bortezomib or parbendazole.

Similarly, and as a second aspect of the present disclosure, there is provided a method of prevention or treatment of dementia comprising administration of bortezomib or parbendazole to a subject in need thereof.

Said dementia of the above aspects is preferably Alzheimer's disease.

The subject of the method of prevention or treatment of the above aspects is preferably human.

When the product of the above aspects is bortezomib, it is preferably given by injection, such as intravenous or subcutaneous injection.

When the product of the above aspects is parbendazole, it is preferably administrated orally, e.g. as a tablet.

**EXAMPLES - METHODS**

All computational analyses were performed on Rstudio version 4.1 for macOS unless otherwise stated.

**Identification of AD druggable target genes**

Hub genes (i.e. genes present in multiple modules and having a high degree in all) from co-expression modules that have been revealed for the dorsolateral prefrontal cortex, temporal cortex and cerebellum were identified using (Bayraktar et al., 2021) data. Significantly up-regulated genes were filtered referring to data from the same study. To determine targetable genes, available knockdown perturbagens for filtered genes were screened from the metadata of signature profiles (cellinfo_beta.txt, siginfo_beta.txt).

The essentialities of the drug target gene and other possibly targetable genes were evaluated using DepMap data (DepMap 22Q1 Public+Score, Chronos) that was filtered for 114 cell lines from the central nervous system and peripheral nervous system lineages and 317 cell lines from seven tissues (skin, lung, kidney, liver, colon, breast, prostate) whose target gene knockdown perturbagens exists. The essentiality scores of a target gene and other possibly targetable genes measured for the nervous system and the essentiality scores of the target gene measured for seven tissues were plotted.

**Drug identification by using drug repositioning**

shRNA gene knockdown and drug-induced perturbagens were attained from the CMap database as GCTX files. They were split into smaller files for each cell line to speed up the calculations (see Code Availability). shRNA-induced perturbagens for the target gene and drug-induced perturbagens from the same cell lines were extracted using the cmapR package (Enache et al., 2017). Correlation matrices, namely drug-shRNA matrices, were constructed by calculating the Spearman correlation coefficients between combinations of drug-perturbed and target-specific shRNA-perturbed perturbagens for each cell line, subsequently filtered for the optimal dose and time point with the highest correlation coefficients.

Drugs with the highest correlations in the top 1% were identified, ranked for each cell line, and termed as top 1% drugs. The Set 1 drugs were determined as the top 1% of drugs from at least six cell lines (n = 4). The Set 2 drugs were determined as the top 1% of drugs from at least two cell lines with the best median rank (n = 4).

Next, the bioavailability of candidate drugs, i.e. absorption, distribution, metabolism and excretion, were investigated using the SwissADME web tool (Daina et al., 2017). In particular, the blood-brain barrier (BBB) permeability of drugs, the P-glycoprotein provided resistance against them and the degradation via CYP were considered when the tool was used .

**Knockdown and drug profile pathway enrichment**

Pathway enrichment was analysed for the shRNA-induced profiles of drug target gene (*GLS*) and drug-induced profiles of candidate drugs (n=8), a subset of best-correlated drugs (n=100) and randomly selected drugs (n=100) in reference to MSigDB hallmark pathways (n=50) and KEGG pathways (n = 347). Pathway gene sets were accessed using the EnrichmentBrowser package (Geistlinger et al., 2016). EntrezIDs and gene symbol conversions were done using the AnnotationDBI package (Pagès et al., 2017). GSEA was performed using the fgsea package (Sergushichev, 2016) with 10000 permutations. Profile pathway enrichments for each profile from different cell lines were combined using metapro package (Yoon et al., 2021); Benjamini-Hochberg adjusted p-values (adj. p-values) were combined by the weighted Fisher's method whereas normalised enrichment scores (NES) were combined by the weighted Z-method. Due to the higher power of the weighted Fisher's method on unassociated p-values (from unassociated experiments), NES with adj. p-value higher than 0.05 were converted to zero, reflecting their low strength. Adjusted p-values (padj) were converted to Reliability as:

$$Reliability = -\log(padj)$$

Pathway enrichments were visualised as bubble plots.

**Network analysis of drug targeting mechanisms**

CMap data for drug annotations, Cheng's PPI dataset and the Human Reference Interactome PPI dataset were downloaded (Cheng et al., 2018; Luck et al., 2020) (Cheng et al., 2018; Luck et al., 2020). Using CMap data, drugs and target proteins were tabulated as the DPI dataset. The DPI dataset and two PPI datasets which have been created again from multiple sources meticulously were integrated to build an interactome (12057 unique DPIs and 262549 PPIs, connecting 4383 compounds and 16996 proteins) allowing for the depiction of deep drug-protein associations.

Interactome was built using the igraph package. After, subnetworks were generated from the interactome separately for each MOA associated with candidate drugs by setting the maximum length of paths as four (drug <=> drug target <=> neighbour of target gene <=> target gene) i) to emphasise the most direct drug-target associations and ii) to reduce computational expenses. These subnetworks were imported to Cytoscape using the RCy3 package for better examination of target protein-drug associations and visualisation (Gustavsen et al., 2019).

**_In vitro_ validation of candidate drug pharmacodynamics**

The toxicity, induced protein level changes and efficacy of two candidate drugs (parbendazole and bortezomib) and a disease reference drug (memantine) were examined on the glioblastoma cell line U138MG. Before performing these assays, the optimum cell count per well was decided by comparing the optical density for L-glutamate (ab120049) absorbance between DMEM-0819 (with glutamine) and DMEM-5671 (without glutamine) culture media, which was tested by glutamate assay kit from Abcam (Abcam PLC, Cambridge, UK) at 450 nm following its protocol, then cultured 20000 cells per well in DMEM-0819 with L-alanyl-L glutamine, adherently, treated with drugs dissolved in DMSO separately. To prepare LDH assay high control samples, cell lysis solution was added instead of drugs. Samples were incubated in a humidified incubator (at 37 °C, 5% CO2). A microplate reader was used to measure absorbances.

Western blot

Drug-induced glutaminase content was measured by western. The cells were washed with PBS and lysed with CelLytic M (C2978, Sigma-Aldrich, Saint Louis, MO, USA) lysis buffer containing protease inhibitors. The lysates were centrifuged at 12,000 rotations per minute for 5 min, and the supernatant was collected. Proteins were separated by Mini-PROTEAN® TGXTM Precast Gels (BioRad, Berkeley, CA, USA) and transferred to a Trans-Blot Turbo Mini 0.2um PVDF Transfer Packs membrane (BioRad, Berkeley, CA, USA) by using Trans-Blot® TurboTM Transfer System (Bio-Rad, Berkeley, CA, USA). The antibodies for mitochondrial glutaminase-1 (kidney-type) and glutaminase-2 (liver-type) encoded from two GLS isomers and GAPDH were used for primary immunoblotting. All the antibodies were diluted at a 1:1000 concentration. DMSO was used as a blank reference. The membranes were incubated in primary antibody solution overnight at 4 °C with gentle rocking. The secondary antibody, goat Anti-Rabbit HRP (ab205718) or goat anti-mouse IgG-HRP (sc2005, Santa Cruz Biotechnology, Inc., Dallas, TX, USA), was blotted for 30 min at 4 °C with gentle rocking. The protein bands were detected with ImageQuant LAS 500 (29-0050-63)

Drug Toxicity

Tetrazolium reduction assay (MTT assays) and lactate dehydrogenase release assays (LDH assays) were conducted to assess cell metabolic activity (cell proliferation) and to measure cytotoxicity (drug toxicity). Abcam protocols (MTT: ab211091, LDH: ab65293) were followed. Here, both protocols were summarized.

Before adding assay solutions on day 1 (24 hours) and day 2 (48 hours), the drug treatment media were aspirated from plates and transferred to well plates.

For the MTT assay, serum-free media and MTT Reagent were added to each well. Control samples were prepared without using cells but serum-free media and MTT reagent. Well plates were incubated at 37°C for 3 hours, MTT Solvent was added to each well, and the wells were wrapped in foil and shaken on an orbital shaker for 15 minutes. The reduction of MTT by mitochondrial succinate dehydrogenase was measured by reading absorbance at OD = 590 nm (yellow to purple). Cytotoxicity (CT) was calculated as a ratio of measures of absorbance of control wells (AbsC) and absorbance of sample-containing wells (AbsS) as below:

$$CT = 100 * \frac{(AbsC - AbsS)}{AbsC}$$

For the LDH release assay, LDH reaction mix was added to wells. Low control samples were prepared only using cells.. Well plates were incubated at 37°C for 30 minutes. The release of LDH from cells due to damaged plasma membrane was measured by reading absorbance at OD = 450 nm (blue). Cytotoxicity (CT) was calculated as a ratio of measures of absorbance of low control wells (AbsLC), high control wells (AbsHC) sample wells (AbsS) as below:

$$CT = 100 * \frac{(AbsS - AbsLC)}{(AbsHC - AbsLC)}$$

Glutamate contents assay

Drug efficacy describes how the drug-protein interaction manipulates the normal action of the protein. Glutamate content was examined at differing drug concentrations with the glutamate assay kit to measure drug efficacies. Abcam protocol (ab83389) was followed to quantitate glutamate content. Here, the protocols were summarized.

To prepare samples, cells were harvested, washed with cold PBS, resuspended in an assay buffer, homogenised, incubated for 15 - 30 min on ice, and centrifuged for 2 - 5 minutes at 4°C at top speed using a cold microcentrifuge. The supernatant was collected and transferred to a clean tube, then distributed to well plates.

To quantitate free glutamate levels, the reaction mixture was added to each standard and sample well. The background reaction mix was added to the background sample wells. Wells were mixed and incubated at 37°C for 30 minutes protected from light. Colour change was measured at OD = 450 nm (blue). Glutamate concentration was calculated as formulated in the protocol, then divided by relative cytotoxicity by MTT assay to extrapolate drug efficacy.

## EXAMPLES - RESULTS

### Identification of AD druggable target genes

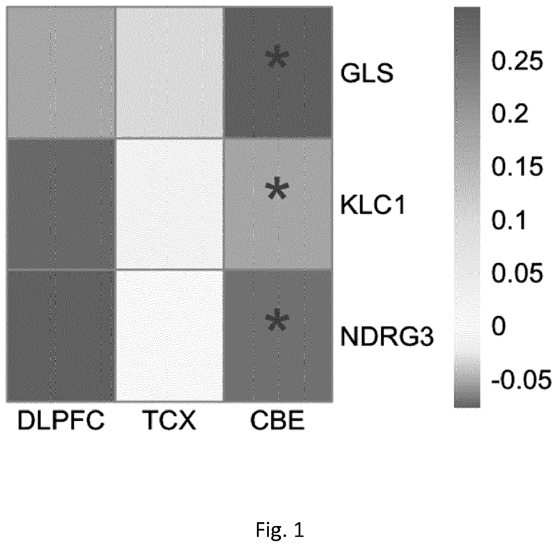

In a previous study, human brain transcriptome profiles from the dorsolateral prefrontal cortex, temporal cortex and cerebellum have been analysed by the use of GCN analysis, showing co-expression patterns of potential pathologydriving genes, which is consistent through the diseased brain (Bayraktar et al., 2021). As a result, it was found that 95 genes were shared by the functional modules derived from multiple brain regions. Furthermore, focus was put on up-regulated genes that could be suppressed by drugs, since drug-induced overexpression is more likely to increase drug resistance which is specifically risky for elderly people (Palmer and Kishony, 2014). Among these 95 genes, only *GLS, KLC1* and *NDRG3* were significantly up-regulated in the AD brain (Figure 1) and only *GLS* had available gene knockdown transcriptome profiles in the CMap database, which could be used for the drug repositioning analysis.

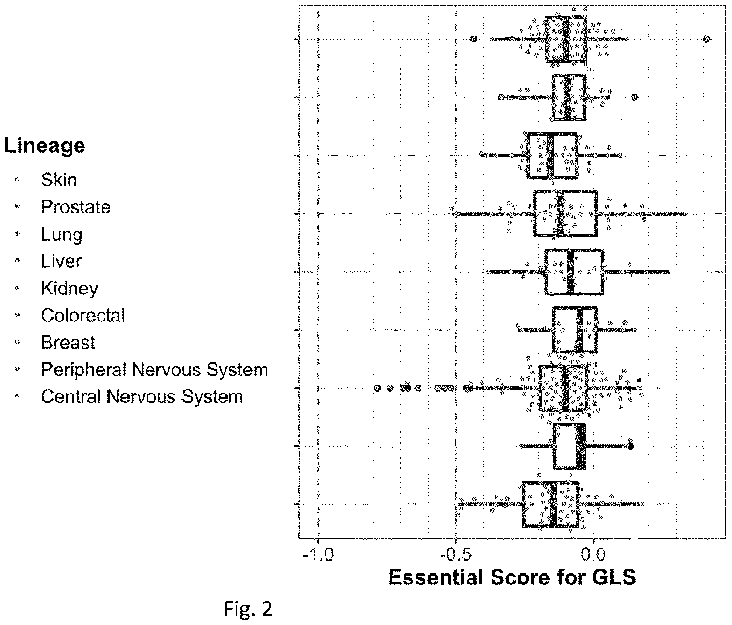

The essentiality of *GLS* in the nervous system was checked to ensure its targetability. Cancer Dependency Map (DepMap) stores extensive genomic information about cancer and regular cell lines screened for CRISPR gene knockdowns. Powerful algorithms are used in the database to deduce gene knockdown fitness effects to estimate genedependent survivability (Dempster et al., 2021; Meyers et al., 2017). Whereas essential genes with low essential scores are targeted in cancer research, a neurodegeneration study should target genes nonessential for cell proliferation with high essential scores hypothetically (Jaladanki et al., 2021). The screening of 114 cell lines of nervous system lineage showed that *GLS* had a high essential score as observed in (Jaladanki et al., 2021), confirming glutaminase targetability in the brain (Figure 2). Supporting this, *GLS* knockdown was not predicted to inhibit cell proliferation necessarily in other tissues (n = 371).

*GLS* encodes glutaminase (i.e., glutaminase-1 or kidney-type glutaminase) that is located in the mitochondrial intermembrane and hydrolyzes glutamine to glutamate and ammonium which is the primary reaction for glutamate biosynthesis (Márquez et al., 2016). Glutamate can be further converted to other amino acids (arginine, aspartate, isoleucine, leucine, serine, valine, tyrosine), $\alpha$-ketoglutarate, which is a member of the citric acid cycle, and glutathione, which is a universal antioxidant (Walker and Donk, 2016). More importantly, glutamate is the most abundant excitatory neurotransmitter, stimulating post-synaptic N-methyl-D-aspartate receptors (NMDARs). Hence, glutamate is vital for protein metabolism, energy production and transmitting information across the central nervous system. Normal glutaminase activity is essential for brain health.

However, excess glutamate release from presynaptic regions and glutamate spillover can activate extrasynaptic NMDARs and presynaptic metabotropic glutamate receptors, disrupting ion balance via Ca++ influx, leading to regression of dendritic spines and reductions in synaptic glutamate transmission, ultimately ending in neuron loss. Glutaminase hyperactivity is neurotoxic due to the increase of glutamate and decrease of glutamine concentrations (Haroon et al., 2017; Rumping et al., 2019). Supporting this, high levels of glutamine and glutamate in the AD brain fuelling the glutamate excitotoxicity based on the reporter metabolite analysis have been reported (Bayraktar et al., 2021). All these pieces of evidence emphasise the danger of glutaminase overexpression in Alzheimer's and the hypothesis that glutaminase suppression and glutaminase-dependent reduction in glutamate may be therapeutic for AD.

**Drug repositioning for GLS**

In our proposed drug repositioning method, it is hypothesised that a drug has an inhibitory effect on the target gene if the drug treatment led to expressional changes highly correlated to expressional changes caused by the inhibition of that target gene (Li et al., 2022b).

In the CMap database, seven available cell lines representing different cancerous tissues were found: skin (A375), lung (A549), kidney (HA1E), liver (HEPG2), colon (HT29), breast (MCF7) and prostate (PC3) with both drug-perturbed and GLS knockdown induced signature profiles. In the drug repositioning analysis, the correlation matrices, denoted as drug-shRNA matrices, were first constructed by calculating the Spearman correlation between drug-perturbed and *GLS* knockdownperturbed signature profiles for each cell line, totally generating seven matrices, and optimised for the optimal dose and time point with the highest correlation for each shRNA-drug combination on each cell line. Drugs were ranked based on correlation coefficients and then the best 1% drugs with the highest correlation for each cell line were extracted. The drugs showing a highly similar effect of *GLS* knockdown on a tissue - medium or strong correlation: > 0.3 (Akoglu, 2018) - would be evaluated. A549 showed the best drug-shRNA profile combinations; the 99th percentile correlation was 0.585 and the maximum correlation for a drug-shRNA was 0.684. Other than HT29 (whose 99th percentile correlation was 0.202 and maximum correlation was 0.343), all selected drug profiles were fairly correlated to *GLS* knockdown profiles (Table 1).

Table 1: correlations of drug-perturbed profiles to target gene knockdown profiles at several thresholds. Interpretations of the correlations go like this: +0.60 (strong), +0.50 (slightly strong), +0.40 (moderate), +0.30 (passable), +0.20 (poor), - 0.20 (negligible).

|  | A375 | A549 | HA1E | HEPG2 | HT29 | MCF7 | PC3 |
|---|---|---|---|---|---|---|---|
| 99th quantile (= top 1%) | 0.381 | 0.585 | 0.464 | 0.326 | 0.201 | 0.306 | 0.312 |
| 99.5th quantile | 0.405 | 0.616 | 0.478 | 0.349 | 0.221 | 0.319 | 0.325 |
| 99.9th quantile | 0.442 | 0.649 | 0.512 | 0.398 | 0.258 | 0.341 | 0.353 |
| Maximum | 0.503 | 0.683 | 0.543 | 0.429 | 0.343 | 0.373 | 0.370 |

Primarily, drugs with the best representation were considered. Mitoxantrone was the only drug represented (at the top 1%) in all seven cell lines, whereas crizotinib, bortezomib and withaferin-a were represented in six cell lines. However, pharmacokinetic models accessible in the SwissADME web tool (Daina et al., 2017) predicted that these anti-cancer drugs were unlikely to reach the brain tissues through the blood-brain barrier (BBB) and more likely to be effluated and degraded by P-glycoproteins and cytochromes P450 (CYP) in the brain, lowering their supposed efficacy.

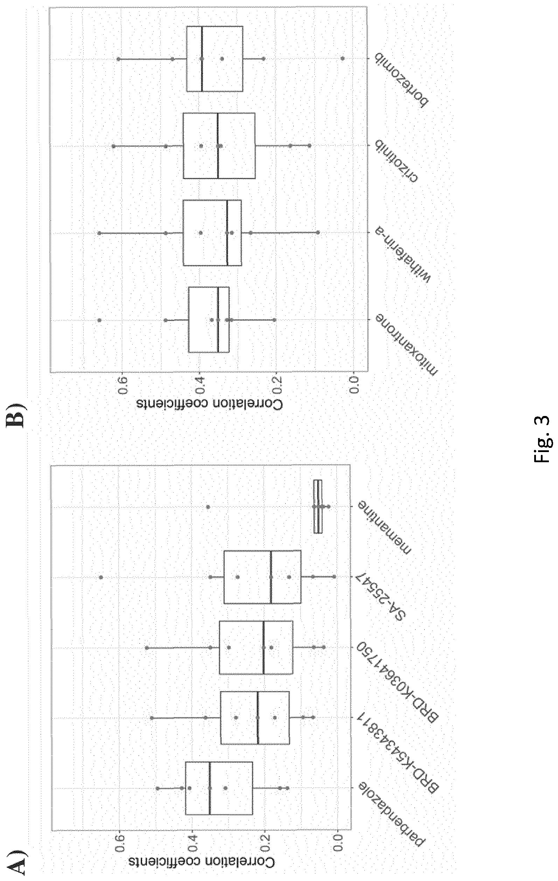

Therefore, an additional set of drugs was gathered by 1) gathering the top 1% of drugs, 2) filtering drugs presented in at least two cell lines (n = 693; likelycorrelated), and 3) getting median ranks. Then, the list was narrowed down to the four best-ranked drugs (parbendazole, BRD-K5433811, BRD-K03641750 and SA-25547), to continue further analyses together with the former four drugs (mitoxantrone, crizotinib, bortezomib, withaferin-a). Only parbendazole was on par with the previously given drugs considering the correlation coefficients (Figure 3). All candidate drugs were screened by the SwissADME tool again for their bioavailability. Though parbendazole, BRD-K5433811 and SA-25547 were predicted to permeate the BBB, BRD-K5433811 was predicted to be effluated from the brain. Detailed pharmacological predictions for these compounds were given in Supplementary Table 1.

Memantine was included in the study as a positive control since it inhibits glutamate receptors weakly and is an often-used drug in AD treatment (Robinson and Keating, 2006). To note, memantine-perturbed profiles were not correlated to GLS knockdown profiles.

**Knockdown and drug-perturbed profile pathway enrichment**

Gene set enrichment analysis (GSEA) was performed on GLS-knockdown and candidate drug profiles to further elaborate on the effect of gene suppression and drug inductions on biological pathways in terms of biological states and processes. Reference was made to pathways in the Kyoto Encyclopaedia of Genes and Genomes knowledge base (KEGG) (Kanehisa and Goto, 2000) and hallmark gene sets from the Molecular Signatures Database (MSigDB) (Liberzon et al., 2015). Genesets from both databases are well-defined and curated; however, have been generated regarding different visions and delineate different biological spaces.

Initially, perturbagens of 100 randomly selected drugs and 100 best-correlated drugs were profiled for hallmark pathways from MSigDB to later be aggregated using the weighted Fisher approach (Yoon et al., 2021), assuming that i) reliable changes in biological pathways on a multi-tissue scale as a response to small molecules could be captured and ii) drugs sharing similar biological effects should also share same pathway enrichment patterns.

It was revealed that energetic metabolism (e.g., oxidative phosphorylation and glycolysis), biosynthetic metabolism (e.g., fatty acid metabolism, adipogenesis, and protein secretion) and cell cycle events (e.g., G2M checkpoint, DNA repair and P53 pathway) were suppressed whereas inflammatory response and various signalling paths (RAS, JAK/STAT and TNFA) were enriched coherently as a response to highly correlated drug inductions. These results suggested that the drugs identified by the approach showed a clear suppression effect on the expression of GLS and also for candidate drug inductions with note-worthy differences. For instance, mitotic spindle (gene set) repression and Ras signalling pathway enrichment were more reliable and stronger on drug inductions compared to gene suppression, emphasising the effect of these drugs on the cell cycle.

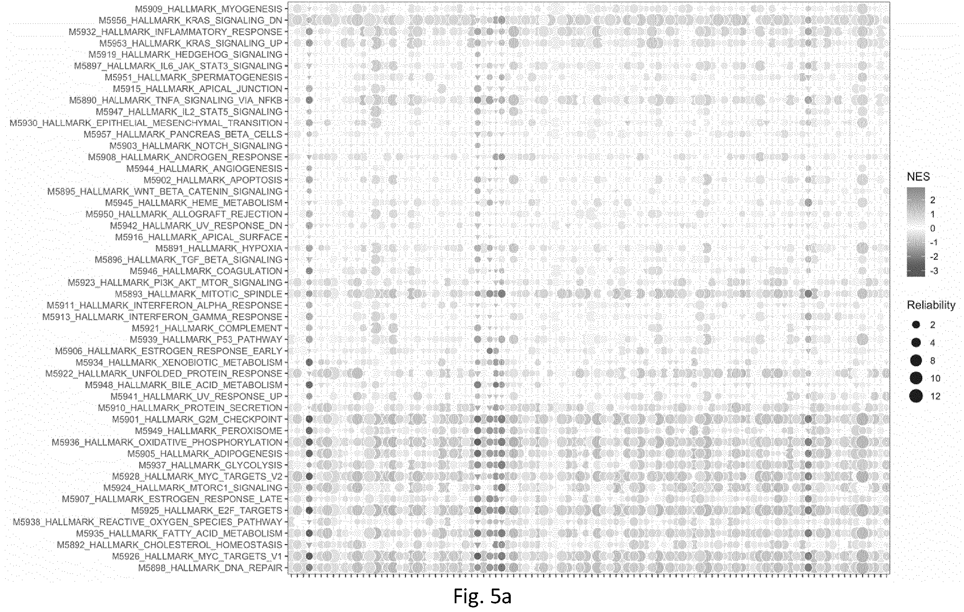

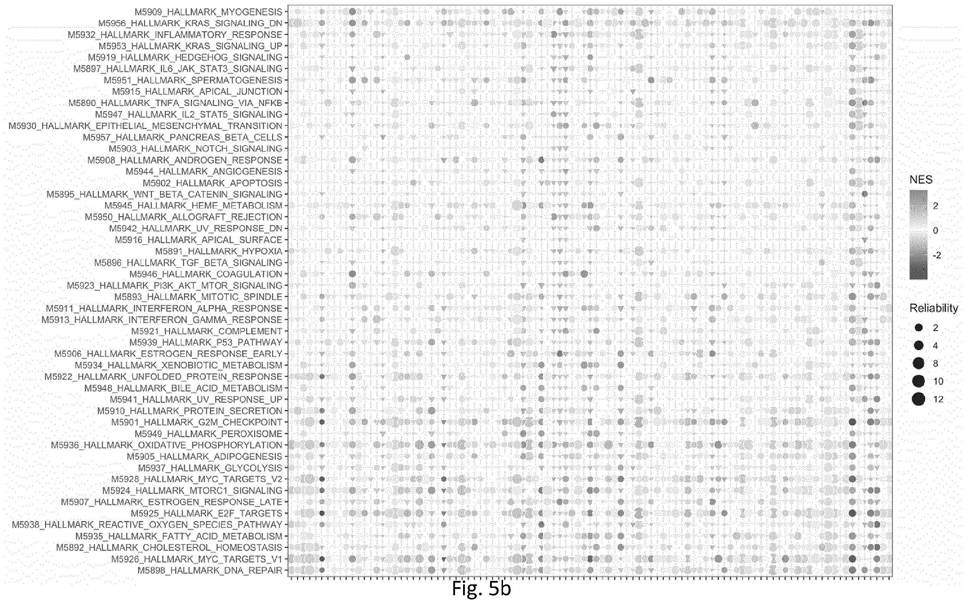

KEGG pathway enrichments added further annotations supporting these patterns, which were often more radical (e.g. calcium and cAMP signalling pathway enrichments) (Figure 4). In contrast, enrichment patterns for randomly given drugs were neither significant, strong nor consistent (Figure 5).

## Network analysis of drug efficacy mechanisms

Thus far, the functional resemblance of candidate drug inductions and glutaminase suppression on biological processes was shown. Nonetheless, causal (i.e. physical) links between molecules need to be shown as well to assure that similar effects do not occur by chance. Network-based approaches can exhibit the physical interactions between drugs and target molecules directly or indirectly in a robust way (Zhou et al., 2020).

Hence, this method was incorporated to augment the understanding of the role of candidate repurposable drugs. CMap Repurposing App (https://clue.io/repurposing-app) provides a curated table of compound metadata including MOAs that they associate, proteins that they bind and clinical indications that they have been used against, displaying such information about candidate drugs. The correlated drugs targeting the same biological entities were listed, which disclosed that they have been in use against diseases ranging from gout and fever to rheumatoid arthritis and myeloma. It appeared that five of these candidate drugs have known MOAs, or in other words, it is evidenced that they target a few biological entities or processes: NFkB signalling pathway, topoisomerase, proteasome and ALK tyrosine kinase.

The CMap database provides physical drug-target interactions, which have been incorporated from various external sources. The drug-target interactions dataset and two extensive protein-protein interaction datasets (Cheng et al., 2018; Luck et al., 2020), which have been created again from multiple sources meticulously, were integrated to build an interactome (12057 unique drug-protein interactions (DPIs) and 262549 protein-protein interactions (PPIs), connecting 4383 compounds and 16996 proteins) allowing for the depiction of deep drug-protein associations.

The inventors then zoomed in on the interactome for each MOA associated with candidate drugs by setting the maximum length of paths as four (drug <=> drug target <=> neighbour of target gene <=> target gene) i) to emphasise the most direct drug-target associations and ii) to reduce computational expenses. Later, these subnetworks were assembled, revealing that glutaminase has been evidenced to interact physically with several proteins responsible for signal-induced membrane trafficking (ATXN10, ARF6, CAPN1, PARD6, MYL12A), transcriptional activation (transcription factor encoded by ATF2) and ubiquitination of targeted proteins (CUL5, UBC, EIF5A). Signal-induced membrane trafficking is complementary to the role of glutamate as the major excitatory neurotransmitter, whereas transcriptional activation and ubiquitination may indicate the regulation of the glutaminase expressions directly. Four candidate drugs interact indirectly with CUL5 or UBC; hence, it can be hypothesised that all four of them may potentially lead to proteolysis of glutaminases.

Proteasomal proteins (PSM family) and NFkB subunit (RELA) interact with UBC and CUL5. From the literature, it is known that the ubiquitin-proteasomal system is a major regulator of protein degradation (Fernández-Fernández et al., 2017). The effects of topoisomerases (TOP2A) are less clear. It is known that they can allow gene expressions by unwinding DNA and they may activate the ubiquitin-proteasomal system for target proteins via inducing NF-kB, two opposing mechanisms (Hande, 2008); however, these proteins are too specific on how they act on their targets that cannot be understood from a non-directed interactome. A similar dichotomy is present for parbendazole-inhibited tubulin (TUBB), which may induce the degradation of glutaminases via CUL5 or activation of their synthesis via ATF2, theoretically.

## In vitro validation of drug efficacies and toxicity

As a next step to verify candidate drugs *in vitro* models, the toxicity, induced protein levels and efficacy of two selected candidate drugs (parbendazole and bortezomib) and memantine as a positive control were assessed on the glioblastoma cell line (U138MG) as given in above.

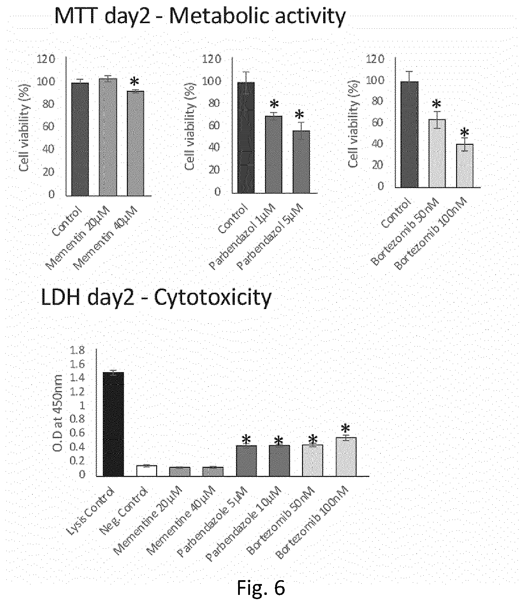

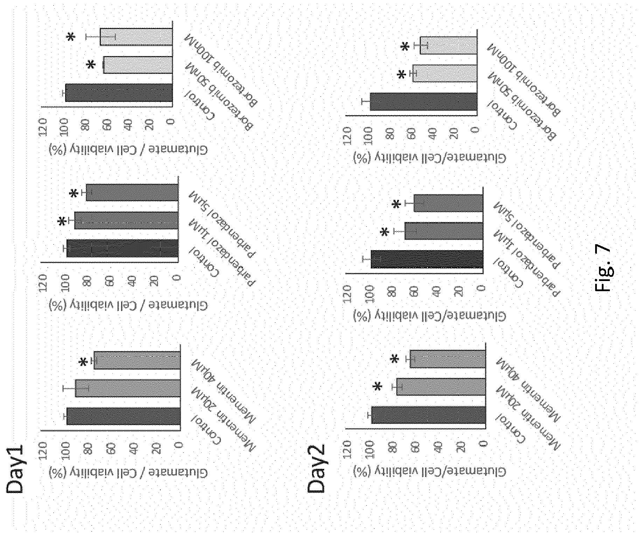

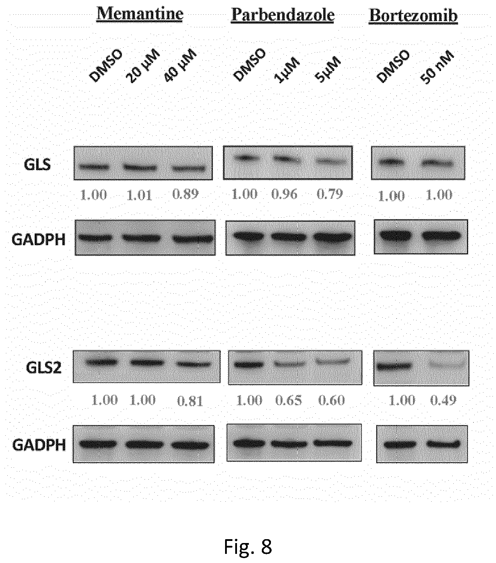

Drug toxicity was considered by MTT and LDH to release assays, which are used to test metabolic activity and cytotoxicity, respectively. Both parbendazole and bortezomib were shown to be toxic for glial cells even at low concentrations, whereas memantine toxicity was insignificant (Figure 6). On the other hand, glutamate levels were highly decreased after the treatment of all three drugs, which are comparable when cell viability was considered (Figure 7). This suggests that parbendazole and bortezomib may show their intended efficacy despite their known anti-apoptotic effects. Western blotting for glutaminase-1 and glutaminase-2 was conducted to measure drug-induced levels for relative band intensities and find a direct or indirect interaction between drug and target as depicted from drug-target network associations. While significant drug affinity for glutaminase-1 could be measured for only comparably higher concentrations of memantine and parbendazole, significant and higher affinity for glutaminase-2 could be measured for all, which were drastic for parbendazole and bortezomib compared to memantine (Figure 8). Consequently, *in vitro* experiments verified the computational predictions of that the repurposed drugs (parbendazole and bortezomib) reduce glutamate levels via suppression of glutaminase.

**REFERENCES**

Akoglu, H. (2018). User's guide to correlation coefficients. Turkish J. Emerg. Med. 18, 91-93.

Al-Hadiya, B.M.H. (2010). Parbendazole. In Profiles of Drug Substances, Excipients and Related Methodology, (Academic Press), pp. 263-284.

Ancidoni, A., Bacigalupo, I., Remoli, G., Lacorte, E., Piscopo, P., Sarti, G., Corbo, M., Vanacore, N., and Canevelli, M. (2021). Anticancer drugs repurposed for Alzheimer's disease: a systematic review. Alzheimer's Res. Ther. 13, 1-15.

Aunan, J.R., Cho, W.C., and Søreide, K. (2017). The biology of aging and cancer: A brief overview of shared and divergent molecular hallmarks. Aging Dis. 8, 628-642.

Bayraktar, A., Lam, S., Altay, O., Li, X., Yuan, M., Zhang, C., Arif, M., Turkez, H., Uhlén, M., Shoaie, S., et al. (2021). Revealing the molecular mechanisms of alzheimer's disease based on network analysis. Int. J. Mol. Sci. 22.

Bonnet, R., Mariault, L., and Peyron, J.F. (2022). Identification of potentially anti-COVID-19 active drugs using the connectivity MAP. PLoS One 17.

Chartier, N., Epstein, J., Soudant, M., Dahan, C., Michaud, M., Pittion-Vouyovitch, S., Guillemin, F., Debouverie, M., and Mathey, G. (2018). Clinical followup of 411 patients with relapsing and progressive multiple sclerosis 10 years after discontinuing mitoxantrone treatment: a real-life cohort study. Eur. J. Neurol. 25, 1439-1445.

Chen, F., Guan, Q., Nie, Z.Y., and Jin, L.J. (2013). Gene expression profile and functional analysis of Alzheimer's disease. Am. J. Alzheimers. Dis. Other Demen. 28, 693-701.

Cheng, F., Desai, R.J., Handy, D.E., Wang, R., Schneeweiss, S., Barabási, A.L., and Loscalzo, J. (2018). Network-based approach to prediction and populationbased validation of in silico drug repurposing. Nat. Commun. 9, 1-12.

Cheng, Z., Wen, Y., Liang, B., Chen, S., Liu, Y., Wang, Z., Cheng, J., Tang, X., Xin, H., and Deng, L. (2019). Gene expression profile-based drug screen identifies SAHA as a novel treatment for NAFLD. Mol. Omi. 15, 50-58.

Choudhury, M., Yin, X., Schaefbauer, K.J., Kang, J.H., Roy, B., Kottom, T.J., Limper, A.H., and Leof, E.B. (2020). SIRT7-mediated modulation of glutaminase 1 regulates TGF-$\beta$-induced pulmonary fibrosis. FASEB J. 34, 8920-8940.

Clough, E., and Barrett, T. (2016). The Gene Expression Omnibus database. In Methods in Molecular Biology, (NIH Public Access), pp. 93-110.

Culhane, A.C., Schröder, M.S., Sultana, R., Picard, S.C., Martinelli, E.N., Kelly, C., Haibe-Kains, B., Kapushesky, M., St Pierre, A.A., Flahive, W., et al. (2012). GeneSigDB: A manually curated database and resource for analysis of gene expression signatures. Nucleic Acids Res. 40, D1060-D1066.

Daina, A., Michielin, O., and Zoete, V. (2017). SwissADME: A free web tool to evaluate pharmacokinetics, drug-likeness and medicinal chemistry friendliness of small molecules. Sci. Rep. 7, 1-13.

Dempster, J.M., Boyle, I., Vazquez, F., Root, D.E., Boehm, J.S., Hahn, W.C., Tsherniak, A., and McFarland, J.M. (2021). Chronos: a cell population dynamics model of CRISPR experiments that improves inference of gene fitness effects. Genome Biol. 22,1-23.

Dezsi, L., and Vecsei, L. (2017). Monoamine Oxidase B Inhibitors in Parkinson's Disease. CNS Neurol. Disord. - Drug Targets 16.

Enache, O.M., Lahr, D.L., Natoli, T.E., Litichevskiy, L., Wadden, D., Flynn, C., Gould, J., Asiedu, J.K., Narayan, R., and Subramanian, A. (2017). The GCTx format and cmap{Py, R, M} packages: resources for the optimized storage and integrated traversal of dense matrices of data and annotations. BioRxiv 227041.

Eshraghi, M., Ahmadi, M., Afshar, S., Lorzadeh, S., Adlimoghaddam, A., Rezvani Jalal, N., West, R., Dastghaib, S., Igder, S., Torshizi, S.R.N., et al. (2022). Enhancing autophagy in Alzheimer's disease through drug repositioning. Pharmacol. Ther. 237, 108171.

Fernández-Fernández, M.R., Gragera, M., Ochoa-Ibarrola, L., Quintana-Gallardo, L., and Valpuesta, J.M. (2017). Hsp70 - a master regulator in protein degradation. FEBS Lett. 591, 2648-2660.

Finberg, J.P.M., and Rabey, J.M. (2016). Inhibitors of MAO-A and MAO-B in psychiatry and neurology. Front. Pharmacol. 7, 340.

Fischer, B. (2017). rhdf5-HDF5 interface for R.

Geistlinger, L., Csaba, G., and Zimmer, R. (2016). Bioconductor's EnrichmentBrowser: Seamless navigation through combined results of set- & network-based enrichment analysis. BMC Bioinformatics 17, 1-11.

Glicksberg, B.S., Li, L., Chen, R., Dudley, J., and Chen, B. (2019). Leveraging big data to transform drug discovery. In Methods in Molecular Biology, (NIH Public Access), pp. 91-118.

Gustavsen, J.A., Pai, S., Isserlin, R., Demchak, B., and Pico, A.R. (2019). RCy3: Network biology using Cytoscape from within R. FioooResearch 8, 1774.

Hande, K.R. (2008). Topoisomerase II inhibitors. Update Cancer Ther. 3, 13-26.

Haroon, E., Miller, A.H., and Sanacora, G. (2017). Inflammation, Glutamate, and Glia: A Trio of Trouble in Mood Disorders. Neuropsychopharmacology 42, 193-215.

Hascup, K.N., Findley, C.A., Britz, J., Esperant-Hilaire, N., Broderick, S.O., Delfino, K., Tischkau, S., Bartke, A., and Hascup, E.R. (2021). Riluzole attenuates glutamatergic tone and cognitive decline in A$\beta$PP/PS1 mice. J. Neurochem. 156, 513-523.

Hu, W., Zhang, C., Wu, R., Sun, Y., Levine, A., and Feng, Z. (2010). Glutaminase 2, a novel p53 target gene regulating energy metabolism and antioxidant function. Proc. Natl. Acad. Sci. U. S. A. 107, 7455-7460.

Imre, G. (2007). Anti-apoptotic and Pro-inflammatory Signaling in Cancer Cells : Status and Modulation by Chemotherapeutic Drugs.

Jaladanki, S.K., Elmas, A., Malave, G.S., and Huang, K. lin (2021). Genetic dependency of Alzheimer's disease-associated genes across cells and tissue types. Sci. Rep. 11, 1-8.

Johmura, Y., Yamanaka, T., Omori, S., Wang, T.W., Sugiura, Y., Matsumoto, M., Suzuki, N., Kumamoto, S., Yamaguchi, K., Hatakeyama, S., et al. (2021). Senolysis by glutaminolysis inhibition ameliorates various age-associated disorders. Science (80-. ). 371, 265-270.

Jourdan, J.P., Bureau, R., Rochais, C., and Dallemagne, P. (2020). Drug repositioning: a brief overview. J. Pharm. Pharmacol. 72, 1145-1151.

Kanehisa, M., and Goto, S. (2000). KEGG: Kyoto Encyclopedia of Genes and Genomes. Nucleic Acids Res. 28, 27-30.

Ko, Y. (2020). Computational drug repositioning: Current progress and challenges. Appl. Sci. 10, 5076.

Lee, I.C., and Choi, B.Y. (2016). Withaferin-A-A natural anticancer agent with pleitropic mechanisms of action. Int. J. Mol. Sci. 17.

Lee, H., Kang, S., and Kim, W. (2016). Drug repositioning for cancer therapy based on large-scale drug-induced transcriptional signatures. PLoS One 11, 00150460.

Li, X., Shong, K., Kim, W., Yuan, M., Yang, H., Sato, Y., Kume, H., Ogawa, S., Turkez, H., Shoaie, S., et al. (2022a). Prediction of drug candidates for clear cell renal cell carcinoma using a systems biology-based drug repositioning approach. 78, 103963.

Li, X., Shong, K., Kim, W., Yuan, M., Yang, H., Sato, Y., Kume, H., Ogawa, S., Turkez, H., Shoaie, S., et al. (2022b). Prediction of drug candidates for clear cell renal cell carcinoma using a systems biology-based drug repositioning approach. EBioMedicine 78, 103963.

Luck, K., Kim, D.K., Lambourne, L., Spirohn, K., Begg, B.E., Bian, W., Brignall, R., Cafarelli, T., Campos-Laborie, F.J., Charloteaux, B., et al. (2020). A reference map of the human binary protein interactome. Nature 580, 402-408.

Manousi, A., Göttle, P., Reiche, L., Cui, Q.L., Healy, L.M., Akkermann, R., Gruchot, J., Schira-Heinen, J., Antel, J.P., Hartung, H.P., et al. (2021). Identification of novel myelin repair drugs by modulation of oligodendroglial differentiation competence. EBioMedicine 65, 103276.

Márquez, J., Matés, J.M., and Campos-Sandoval, J.A. (2016). Glutaminases. Adv. Neurobiol. 13, 133-171.

Matés, J.M., Campos-sandoval, J.A., Santos-jiménez, J.D.L., Segura, J.A., Alonso, F.J., and Márquez, J. (2019). Metabolic Reprogramming of Cancer by Chemicals that Target Glutaminase Metabolic Reprogramming of Cancer by Chemicals that Target Glutaminase Isoenzymes.

Matthews, D.C., Mao, X., Dowd, K., Tsakanikas, D., Jiang, C.S., Meuser, C., Andrews, R.D., Lukic, A.S., Lee, J., Hampilos, N., et al. (2021). Riluzole, a glutamate modulator, slows cerebral glucose metabolism decline in patients with Alzheimer's disease. Brain 144, 3742-3755.

Meyers, R.M., Bryan, J.G., McFarland, J.M., Weir, B.A., Sizemore, A.E., Xu, H., Dharia, N. V., Montgomery, P.G., Cowley, G.S., Pantel, S., et al. (2017). Computational correction of copy number effect improves specificity of CRISPR-Cas9 essentiality screens in cancer cells. Nat. Genet. 49, 1779-1784.

Mullane, K., and Williams, M. (2020). Alzheimer's disease beyond amyloid: Can the repetitive failures of amyloid-targeted therapeutics inform future approaches to dementia drug discovery? Biochem. Pharmacol. 177, 113945.

Nevado-Holgado, A.J., and Lovestone, S. (2017). Determining the Molecular Pathways Underlying the Protective Effect of Non-Steroidal AntiInflammatory Drugs for Alzheimer's Disease: A Bioinformatics Approach. Comput. Struct. Biotechnol. J. 15, 1-7.

Pagès, H., Carlson, M., Falcon, S., and Maintainer, N.L. (2017). Package 'AnnotationDbi.' Bioconductor Packag. Maint.

Palmer, A.C., and Kishony, R. (2014). Opposing effects of target overexpression reveal drug mechanisms. Nat. Commun. 5, 1-8.

Rangaraju, S., Dammer, E.B., Raza, S.A., Rathakrishnan, P., Xiao, H., Gao, T., Duong, D.M., Pennington, M.W., Lah, J.J., Seyfried, N.T., et al. (2018). Identification and therapeutic modulation of a pro-inflammatory subset of disease-associated-microglia in Alzheimer's disease. Mol. Neurodegener. 13, 1-25.

Robinson, D.M., and Keating, G.M. (2006). Memantine: A review of its use in Alzheimer's disease. Drugs 66, 1515-1534.

Rumping, L., Tessadori, F., Pouwels, P.J.W., Vringer, E., Wijnen, J.P., Bhogal, A.A., Savelberg, S.M.C., Duran, K.J., Bakkers, M.J.G., Ramos, R.J.J., et al. (2019). GLS hyperactivity causes glutamate excess, infantile cataract and profound developmental delay. Hum. Mol. Genet. 28, 96-104.

Sergushichev, A.A. (2016). An algorithm for fast preranked gene set enrichment analysis using cumulative statistic calculation. BioRxiv 060012.

Sharma, S.K. (2010). Hepatocyte Growth Factor in Synaptic Plasticity and Alzheimer ' s Disease. Sci. World J. 10, 457-461.

Shaw, A.T., Yasothan, U., and Kirkpatrick, P. (2011). Crizotinib. Nat. Rev. Drug Discov. 10, 897-898.

Sikora, E., Bielak-Zmijewska, A., Dudkowska, M., Krzystyniak, A., Mosieniak, G., Wesierska, M., and Wlodarczyk, J. (2021). Cellular Senescence in Brain Aging. Front. Aging Neurosci. 13, 1-23.

Subramanian, A., Narayan, R., Corsello, S.M., Peck, D.D., Natoli, T.E., Lu, X., Gould, J., Davis, J.F., Tubelli, A.A., Asiedu, J.K., et al. (2017). A Next Generation Connectivity Map: L1000 Platform and the First 1,000,000 Profiles. Cell 171, 1437-1452.e17.

Trudeau, L.E., and El Mestikawy, S. (2018). Glutamate cotransmission in cholinergic, GABAergic and monoamine systems: Contrasts and commonalities. Front. Neural Circuits 12, 113.

Vossel, K., Ranasinghe, K.G., Beagle, A.J., La, A., Ah Pook, K., Castro, M., Mizuiri, D., Honma, S.M., Venkateswaran, N., Koestler, M., et al. (2021). Effect of Levetiracetam on Cognition in Patients with Alzheimer Disease with and without Epileptiform Activity: A Randomized Clinical Trial. JAMA Neurol. 78, 1345-1354.

Walker, M.C., and Donk, W.A. van der (2016). The Many Roles of Glutamate in Metabolism. Physiol. Behav. 43, 419-430.

Williams, G., Gatt, A., Clarke, E., Corcoran, J., Doherty, P., Chambers, D., and Ballard, C. (2019). Drug repurposing for Alzheimer's disease based on transcriptional profiling of human iPSC-derived cortical neurons. Transl. Psychiatry 9, 1-10.

Wrasidlo, W., Crews, L.A., Tsigelny, I.F., Stocking, E., Kouznetsova, V.L., Price, D., Paulino, A., Gonzales, T., Overk, C.R., Patrick, C., et al. (2014). Neuroprotective effects of the anti-cancer drug sunitinib in models of HIV neurotoxicity suggests potential for the treatment of neurodegenerative disorders. Br. J. Pharmacol. 171, 5757-5773.

Wu, C.H., Apweiler, R., Bairoch, A., Natale, D.A., Barker, W.C., Boeckmann, B., Ferro, S., Gasteiger, E., Huang, H., Lopez, R., et al. (2006). The Universal Protein Resource (UniProt): an expanding universe of protein information. Nucleic Acids Res. 34, D187-D191.

Xiang, L., Mou, J., Shao, B., Wei, Y., Liang, H., Takano, N., Semenza, G.L., and Xie, G. (2019). Glutaminase 1 expression in colorectal cancer cells is induced by hypoxia and required for tumor growth, invasion, and metastatic colonization. Cell Death Dis. 10, 1-15.

Yamamoto, S., and Egashira, N. (2021). Pathological mechanisms of bortezomib-induced peripheral neuropathy. Int. J. Mol. Sci. 22, 1-14.

Yoon, S., Baik, B., Park, T., and Nam, D. (2021). Powerful p-value combination methods to detect incomplete association. Sci. Rep. 11, 1-11.

Yuan, M., Shong, K., Li, X., Ashraf, S., Shi, M., Kim, W., Nielsen, J., Turkez, H., Shoaie, S., Uhlen, M., et al. (2022). A Gene Co-Expression Network-Based Drug Repositioning Approach Identifies Candidates for Treatment of Hepatocellular Carcinoma. Cancers (Basel). 14, 1573.

Zhou, Y., Hou, Y., Shen, J., Huang, Y., Martin, W., and Cheng, F. (2020). Network-based drug repurposing for novel coronavirus 2019-nCoV/SARS-CoV-2. Cell Discov. 6, 14.

**Claims**

1. A product for use in a method of prevention or treatment of dementia, wherein the product is or bortezomib or parbendazole.

2. The product for use according to claim 1, wherein said dementia is Alzheimer's disease.

3. A method of prevention or treatment of dementia comprising administration of bortezomib or parbendazole to a subject in need thereof.

4. The method of claim 3, wherein said dementia is Alzheimer's disease.

Fig. 1

Fig. 2

A)

B)

Fig. 3

Fig. 4a

Fig. 4b

Fig. 5a

Fig. 5b

MTT day2 - Metabolic activity

LDH day2 - Cytotoxicity

Fig. 6

Fig. 7

Fig. 8

# EUROPEAN SEARCH REPORT

Application Number

EP 23 16 0766

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/113363 A1 (NEURODIAGNOSTICS LLC [US]) 13 June 2019 (2019-06-13) * page 4, lines 14-17; claim 20 * | 1-4 | INV. A61K31/4184 A61K31/69 A61K38/00 A61P25/28 |
| Y | SANTOSH CHAUHAN ET AL: "Pharmaceutical screen identifies novel target processes for activation of autophagy with a broad translational potential", NATURE COMMUNICATIONS, vol. 6, no. 1, 27 October 2015 (2015-10-27), XP055577961, DOI: 10.1038/ncomms9620 * page 10, left-hand column, paragraph 3 * | 1-4 | |
| Y | Sharma Satyavan ET AL: "Benzimidazoles" In: "Approaches to Design and Synthesis of Antiparasitic Drugs", 1 January 1997 (1997-01-01), Elsevier, XP093044749, ISSN: 0165-7208 ISBN: 978-0-444-89476-2 vol. 25, pages 195-238, DOI: 10.1016/S0165-7208(97)80030-X, Retrieved from the Internet: URL:http://dx.doi.org/10.1016/S0165-7208(97)80030-X> * table 1 * | 1-4 | |

TECHNICAL FIELDS SEARCHED (IPC)

A61K
A61P

The present search report has been drawn up for all claims

| Place of search | Date of completion of the search | Examiner |
|---|---|---|
| Munich | 9 May 2023 | Lo Giudice, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 16 0766

This annex lists the patent family members relating to the patent documents cited in the above-mentioned European search report.
The members are as contained in the European Patent Office EDP file on
The European Patent Office is in no way liable for these particulars which are merely given for the purpose of information.

09-05-2023

| Patent document cited in search report | | Publication date | Patent family member(s) | | Publication date |
|---|---|---|---|---|---|
| WO 2019113363 | A1 | 13-06-2019 | CN | 111699386 A | 22-09-2020 |
| | | | EP | 3735587 A1 | 11-11-2020 |
| | | | EP | 3980560 A1 | 13-04-2022 |
| | | | JP | 2021511067 A | 06-05-2021 |
| | | | JP | 2023060336 A | 27-04-2023 |
| | | | KR | 20200096582 A | 12-08-2020 |
| | | | US | 2019323083 A1 | 24-10-2019 |
| | | | WO | 2019113363 A1 | 13-06-2019 |
| | | | WO | 2020247591 A1 | 10-12-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **AKOGLU, H.** User's guide to correlation coefficients. *Turkish J. Emerg. Med.,* 2018, vol. 18, 91-93
- Parbendazole. **AL-HADIYA, B.M.H.** Profiles of Drug Substances, Excipients and Related Methodology. Academic Press, 2010, 263-284
- **ANCIDONI, A. ; BACIGALUPO, I. ; REMOLI, G. ; LACORTE, E. ; PISCOPO, P. ; SARTI, G. ; CORBO, M. ; VANACORE, N. ; CANEVELLI, M.** Anticancer drugs repurposed for Alzheimer's disease: a systematic review. *Alzheimer's Res. Ther.,* 2021, vol. 13, 1-15
- **AUNAN, J.R. ; CHO, W.C. ; SØREIDE, K.** The biology of aging and cancer: A brief overview of shared and divergent molecular hallmarks. *Aging Dis.,* 2017, vol. 8, 628-642
- **BAYRAKTAR, A. ; LAM, S. ; ALTAY, O. ; LI, X. ; YUAN, M. ; ZHANG, C. ; ARIF, M. ; TURKEZ, H. ; UHLÉN, M. ; SHOAIE, S. et al.** Revealing the molecular mechanisms of alzheimer's disease based on network analysis. *Int. J. Mol. Sci.,* 2021, vol. 22
- **BONNET, R. ; MARIAULT, L. ; PEYRON, J.F.** Identification of potentially anti-COVID-19 active drugs using the connectivity MAP. *PLoS One,* 2022, vol. 17
- **CHARTIER, N. ; EPSTEIN, J. ; SOUDANT, M. ; DAHAN, C. ; MICHAUD, M. ; PITTION-VOUYOVITCH, S. ; GUILLEMIN, F. ; DEBOUVERIE, M. ; MATHEY, G.** Clinical followup of 411 patients with relapsing and progressive multiple sclerosis 10 years after discontinuing mitoxantrone treatment: a real-life cohort study. *Eur. J. Neurol.,* 2018, vol. 25, 1439-1445
- **CHEN, F. ; GUAN, Q. ; NIE, Z.Y. ; JIN, L.J.** Gene expression profile and functional analysis of Alzheimer's disease. *Am. J. Alzheimers. Dis. Other Demen.,* 2013, vol. 28, 693-701
- **CHENG, F. ; DESAI, R.J. ; HANDY, D.E. ; WANG, R. ; SCHNEEWEISS, S. ; BARABÁSI, A.L. ; LOSCALZO, J.** Network-based approach to prediction and populationbased validation of in silico drug repurposing. *Nat. Commun.,* 2018, vol. 9, 1-12
- **CHENG, Z. ; WEN, Y. ; LIANG, B. ; CHEN, S. ; LIU, Y. ; WANG, Z. ; CHENG, J. ; TANG, X. ; XIN, H. ; DENG, L.** Gene expression profile-based drug screen identifies SAHA as a novel treatment for NAFLD. *Mol. Omi.,* 2019, vol. 15, 50-58

- **CHOUDHURY, M. ; YIN, X. ; SCHAEFBAUER, K.J. ; KANG, J.H. ; ROY, B. ; KOTTOM, T.J. ; LIMPER, A.H. ; LEOF, E.B.** SIRT7-mediated modulation of glutaminase 1 regulates TGF-β-induced pulmonary fibrosis. *FASEB J.,* 2020, vol. 34, 8920-8940
- **CLOUGH, E. ; BARRETT, T.** The Gene Expression Omnibus database. *Methods in Molecular Biology,* 2016, 93-110
- **CULHANE, A.C. ; SCHRÖDER, M.S. ; SULTANA, R. ; PICARD, S.C. ; MARTINELLI, E.N. ; KELLY, C. ; HAIBE-KAINS, B. ; KAPUSHESKY, M. ; ST PIERRE, A.A. ; FLAHIVE, W. et al.** GeneSigDB: A manually curated database and resource for analysis of gene expression signatures. *Nucleic Acids Res.,* 2012, vol. 40, D1060-D1066
- **DAINA, A. ; MICHIELIN, O. ; ZOETE, V.** SwissADME: A free web tool to evaluate pharmacokinetics, drug-likeness and medicinal chemistry friendliness of small molecules. *Sci. Rep.,* 2017, vol. 7, 1-13
- **DEMPSTER, J.M. ; BOYLE, I. ; VAZQUEZ, F. ; ROOT, D.E. ; BOEHM, J.S. ; HAHN, W.C. ; TSHERNIAK, A. ; MCFARLAND, J.M.** Chronos: a cell population dynamics model of CRISPR experiments that improves inference of gene fitness effects. *Genome Biol.,* 2021, vol. 22, 1-23
- **DEZSI, L. ; VECSEI, L.** Monoamine Oxidase B Inhibitors in Parkinson's Disease. *CNS Neurol. Disord. - Drug Targets,* 2017, vol. 16
- **ENACHE, O.M. ; LAHR, D.L. ; NATOLI, T.E. ; LITICHEVSKIY, L. ; WADDEN, D. ; FLYNN, C. ; GOULD, J. ; ASIEDU, J.K. ; NARAYAN, R. ; SUBRAMANIAN, A.** The GCTx format and cmap{Py, R, M} packages: resources for the optimized storage and integrated traversal of dense matrices of data and annotations. *BioRxiv 227041,* 2017
- **ESHRAGHI, M. ; AHMADI, M. ; AFSHAR, S. ; LORZADEH, S. ; ADLIMOGHADDAM, A. ; REZVANI JALAL, N. ; WEST, R. ; DASTGHAIB, S. ; IGDER, S. ; TORSHIZI, S.R.N. et al.** Enhancing autophagy in Alzheimer's disease through drug repositioning. *Pharmacol. Ther.,* 2022, vol. 237, 108171
- **FERNÁNDEZ-FERNÁNDEZ, M.R. ; GRAGERA, M. ; OCHOA-IBARROLA, L. ; QUINTANA-GALLARDO, L. ; VALPUESTA, J.M.** Hsp70 - a master regulator in protein degradation. *FEBS Lett.,* 2017, vol. 591, 2648-2660

- **FINBERG, J.P.M. ; RABEY, J.M.** Inhibitors of MAO-A and MAO-B in psychiatry and neurology. *Front. Pharmacol,* 2016, vol. 7, 340
- **FISCHER, B.** *rhdf5-HDF5 interface for R,* 2017
- **GEISTLINGER, L. ; CSABA, G. ; ZIMMER, R.** Bioconductor's EnrichmentBrowser: Seamless navigation through combined results of set- & network-based enrichment analysis. *BMC Bioinformatics,* 2016, vol. 17, 1-11
- **GLICKSBERG, B.S. ; LI, L. ; CHEN, R. ; DUDLEY, J. ; CHEN, B.** Leveraging big data to transform drug discovery. *Methods in Molecular Biology,* 2019, 91-118
- **GUSTAVSEN, J.A. ; PAI, S. ; ISSERLIN, R. ; DEM-CHAK, B. ; PICO, A.R.** RCy3: Network biology using Cytoscape from within R. *FioooResearch,* 2019, vol. 8, 1774
- **HANDE, K.R.** Topoisomerase II inhibitors. *Update Cancer Ther,* 2008, vol. 3, 13-26
- **HAROON, E. ; MILLER, A.H. ; SANACORA, G.** Inflammation, Glutamate, and Glia: A Trio of Trouble in Mood Disorders. *Neuropsychopharmacology,* 2017, vol. 42, 193-215
- **HASCUP, K.N. ; FINDLEY, C.A. ; BRITZ, J. ; ESPE-RANT-HILAIRE, N. ; BRODERICK, S.O. ; DELFI-NO, K. ; TISCHKAU, S. ; BARTKE, A. ; HASCUP, E.R.** Riluzole attenuates glutamatergic tone and cognitive decline in AβPP/PS1 mice. *J. Neurochem.,* 2021, vol. 156, 513-523
- **HU, W. ; ZHANG, C. ; WU, R. ; SUN, Y. ; LEVINE, A. ; FENG, Z.** Glutaminase 2, a novel p53 target gene regulating energy metabolism and antioxidant function. *Proc. Natl. Acad. Sci. U. S. A.,* 2010, vol. 107, 7455-7460
- **IMRE, G.** *Anti-apoptotic and Pro-inflammatory Signaling in Cancer Cells : Status and Modulation by Chemotherapeutic Drugs,* 2007
- **JALADANKI, S.K. ; ELMAS, A. ; MALAVE, G.S. ; HUANG, K. LIN.** Genetic dependency of Alzheimer's disease-associated genes across cells and tissue types. *Sci. Rep.,* 2021, vol. 11, 1-8
- **JOHMURA, Y. ; YAMANAKA, T. ; OMORI, S. ; WANG, T.W. ; SUGIURA, Y. ; MATSUMOTO, M. ; SUZUKI, N. ; KUMAMOTO, S. ; YAMAGUCHI, K. ; HATAKEYAMA, S. et al.** Senolysis by glutaminolysis inhibition ameliorates various age-associated disorders. *Science,* 2021, vol. 371 (80), 265-270
- **JOURDAN, J.P. ; BUREAU, R. ; ROCHAIS, C. ; DALLEMAGNE, P.** Drug repositioning: a brief overview. *J. Pharm. Pharmacol.,* 2020, vol. 72, 1145-1151
- **KANEHISA, M. ; GOTO, S.** KEGG: Kyoto Encyclopedia of Genes and Genomes. *Nucleic Acids Res.,* 2000, vol. 28, 27-30
- **KO, Y.** Computational drug repositioning: Current progress and challenges. *Appl. Sci.,* 2020, vol. 10, 5076
- **LEE, I.C. ; CHOI, B.Y.** Withaferin-A-A natural anti-cancer agent with pleitropic mechanisms of action. *Int. J. Mol. Sci.,* 2016, vol. 17
- **LEE, H. ; KANG, S. ; KIM, W.** Drug repositioning for cancer therapy based on large-scale drug-induced transcriptional signatures. *PLoS One,* 2016, vol. 11, 00150460
- **LI, X. ; SHONG, K. ; KIM, W. ; YUAN, M. ; YANG, H. ; SATO, Y. ; KUME, H. ; OGAWA, S. ; TURKEZ, H. ; SHOAIE, S. et al.** *Prediction of drug candidates for clear cell renal cell carcinoma using a systems biology-based drug repositioning approach,* 2022, vol. 78, 103963
- **LI, X. ; SHONG, K. ; KIM, W. ; YUAN, M. ; YANG, H. ; SATO, Y. ; KUME, H. ; OGAWA, S. ; TURKEZ, H. ; SHOAIE, S. et al.** Prediction of drug candidates for clear cell renal cell carcinoma using a systems biology-based drug repositioning approach. *EBioMedicine,* 2022, vol. 78, 103963
- **LUCK, K. ; KIM, D.K. ; LAMBOURNE, L. ; SPIROHN, K. ; BEGG, B.E. ; BIAN, W. ; BRIG-NALL, R. ; CAFARELLI, T. ; CAMPOS-LABORIE, F.J. ; CHARLOTEAUX, B. et al.** A reference map of the human binary protein interactome. *Nature,* 2020, vol. 580, 402-408
- **MANOUSI, A. ; GÖTTLE, P. ; REICHE, L. ; CUI, Q.L. ; HEALY, L.M. ; AKKERMANN, R. ; GRU-CHOT, J. ; SCHIRA-HEINEN, J. ; ANTEL, J.P. ; HARTUNG, H.P. et al.** Identification of novel myelin repair drugs by modulation of oligodendroglial differentiation competence. *EBioMedicine,* 2021, vol. 65, 103276
- **MÁRQUEZ, J. ; MATÉS, J.M. ; CAMPOS-SAN-DOVAL, J.A.** Glutaminases. *Adv. Neurobiol.,* 2016, vol. 13, 133-171
- **MATÉS, J.M. ; CAMPOS-SANDOVAL, J.A. ; SAN-TOS-JIMÉNEZ, J.D.L. ; SEGURA, J.A. ; ALONSO, F.J. ; MÁRQUEZ, J.** *Metabolic Reprogramming of Cancer by Chemicals that Target Glutaminase Metabolic Reprogramming of Cancer by Chemicals that Target Glutaminase Isoenzymes,* 2019
- **MATTHEWS, D.C. ; MAO, X. ; DOWD, K. ; TSAKANIKAS, D. ; JIANG, C.S. ; MEUSER, C. ; ANDREWS, R.D. ; LUKIC, A.S. ; LEE, J. ; HAMPI-LOS, N. et al.** Riluzole, a glutamate modulator, slows cerebral glucose metabolism decline in patients with Alzheimer's disease. *Brain,* 2021, vol. 144, 3742-3755
- **MEYERS, R.M. ; BRYAN, J.G. ; MCFARLAND, J.M. ; WEIR, B.A. ; SIZEMORE, A.E. ; XU, H. ; DHARIA, N. V. ; MONTGOMERY, P.G. ; COWLEY, G.S. ; PANTEL, S. et al.** Computational correction of copy number effect improves specificity of CRISPR-Cas9 essentiality screens in cancer cells. *Nat. Genet.,* 2017, vol. 49, 1779-1784

- **MULLANE, K. ; WILLIAMS, M.** Alzheimer's disease beyond amyloid: Can the repetitive failures of amyloid-targeted therapeutics inform future approaches to dementia drug discovery?. *Biochem. Pharmacol.,* 2020, vol. 177, 113945
- **NEVADO-HOLGADO, A.J. ; LOVESTONE, S.** Determining the Molecular Pathways Underlying the Protective Effect of Non-Steroidal AntiInflammatory Drugs for Alzheimer's Disease: A Bioinformatics Approach. *Comput. Struct. Biotechnol. J.,* 2017, vol. 15, 1-7
- **PAGÈS, H. ; CARLSON, M. ; FALCON, S. ; MAINTAINER, N.L.** Package 'AnnotationDbi. *Bioconductor Packag. Maint.,* 2017
- **PALMER, A.C. ; KISHONY, R.** Opposing effects of target overexpression reveal drug mechanisms. *Nat. Commun,* 2014, vol. 5, 1-8
- **RANGARAJU, S. ; DAMMER, E.B. ; RAZA, S.A. ; RATHAKRISHNAN, P. ; XIAO, H. ; GAO, T. ; DUONG, D.M. ; PENNINGTON, M.W. ; LAH, J.J. ; SEYFRIED, N.T. et al.** Identification and therapeutic modulation of a pro-inflammatory subset of disease-associated-microglia in Alzheimer's disease. *Mol. Neurodegener.,* 2018, vol. 13, 1-25
- **ROBINSON, D.M. ; KEATING, G.M.** Memantine: A review of its use in Alzheimer's disease. *Drugs,* 2006, vol. 66, 1515-1534
- **RUMPING, L. ; TESSADORI, F. ; POUWELS, P.J.W. ; VRINGER, E. ; WIJNEN, J.P. ; BHOGAL, A.A. ; SAVELBERG, S.M.C. ; DURAN, K.J. ; BAKKERS, M.J.G. ; RAMOS, R.J.J. et al.** GLS hyperactivity causes glutamate excess, infantile cataract and profound developmental delay. *Hum. Mol. Genet.,* 2019, vol. 28, 96-104
- **SERGUSHICHEV, A.A.** An algorithm for fast preranked gene set enrichment analysis using cumulative statistic calculation. *BioRxiv 060012,* 2016
- **SHARMA, S.K.** Hepatocyte Growth Factor in Synaptic Plasticity and Alzheimer's Disease. *Sci. World J.,* 2010, vol. 10, 457-461
- **SHAW, A.T. ; YASOTHAN, U. ; KIRKPATRICK, P.** Crizotinib. *Nat. Rev. Drug Discov.,* 2011, vol. 10, 897-898
- **SIKORA, E. ; BIELAK-ZMIJEWSKA, A. ; DUDKOWSKA, M. ; KRZYSTYNIAK, A. ; MOSIENIAK, G. ; WESIERSKA, M. ; WLODARCZYK, J.** Cellular Senescence in Brain Aging. *Front. Aging Neurosci.,* 2021, vol. 13, 1-23
- **SUBRAMANIAN, A. ; NARAYAN, R. ; CORSELLO, S.M. ; PECK, D.D. ; NATOLI, T.E. ; LU, X. ; GOULD, J. ; DAVIS, J.F. ; TUBELLI, A.A. ; ASIEDU, J.K. et al.** A Next Generation Connectivity Map: L1000 Platform and the First 1,000,000 Profiles. *Cell,* 2017, vol. 171, 1437-1452.e17
- **TRUDEAU, L.E. ; EL MESTIKAWY, S.** Glutamate cotransmission in cholinergic, GABAergic and monoamine systems: Contrasts and commonalities. *Front. Neural Circuits,* 2018, vol. 12, 113
- **VOSSEL, K. ; RANASINGHE, K.G. ; BEAGLE, A.J. ; LA, A. ; AH POOK, K. ; CASTRO, M. ; MIZUIRI, D. ; HONMA, S.M. ; VENKATESWARAN, N. ; KOESTLER, M. et al.** Effect of Levetiracetam on Cognition in Patients with Alzheimer Disease with and without Epileptiform Activity: A Randomized Clinical Trial. *JAMA Neurol.,* 2021, vol. 78, 1345-1354
- **WALKER, M.C. ; DONK, W.A. VAN DER.** The Many Roles of Glutamate in Metabolism. *Physiol. Behav.,* 2016, vol. 43, 419-430
- **WILLIAMS, G. ; GATT, A. ; CLARKE, E. ; CORCORAN, J. ; DOHERTY, P. ; CHAMBERS, D. ; BALLARD, C.** Drug repurposing for Alzheimer's disease based on transcriptional profiling of human iPSC-derived cortical neurons. *Transl. Psychiatry,* 2019, vol. 9, 1-10
- **WRASIDLO, W. ; CREWS, L.A. ; TSIGELNY, I.F. ; STOCKING, E. ; KOUZNETSOVA, V.L. ; PRICE, D. ; PAULINO, A. ; GONZALES, T. ; OVERK, C.R. ; PATRICK, C. et al.** Neuroprotective effects of the anti-cancer drug sunitinib in models of HIV neurotoxicity suggests potential for the treatment of neurodegenerative disorders. *Br. J. Pharmacol.,* 2014, vol. 171, 5757-5773
- **WU, C.H. ; APWEILER, R. ; BAIROCH, A. ; NATALE, D.A. ; BARKER, W.C. ; BOECKMANN, B. ; FERRO, S. ; GASTEIGER, E. ; HUANG, H. ; LOPEZ, R. et al.** The Universal Protein Resource (UniProt): an expanding universe of protein information. *Nucleic Acids Res.,* 2006, vol. 34, D187-D191
- **XIANG, L. ; MOU, J. ; SHAO, B. ; WEI, Y. ; LIANG, H. ; TAKANO, N. ; SEMENZA, G.L. ; XIE, G.** Glutaminase 1 expression in colorectal cancer cells is induced by hypoxia and required for tumor growth, invasion, and metastatic colonization. *Cell Death Dis,* 2019, vol. 10, 1-15
- **YAMAMOTO, S. ; EGASHIRA, N.** Pathological mechanisms of bortezomib-induced peripheral neuropathy. *Int. J. Mol. Sci.,* 2021, vol. 22, 1-14
- **YOON, S. ; BAIK, B. ; PARK, T. ; NAM, D.** Powerful p-value combination methods to detect incomplete association. *Sci. Rep.,* 2021, vol. 11, 1-11
- **YUAN, M. ; SHONG, K. ; LI, X. ; ASHRAF, S. ; SHI, M. ; KIM, W. ; NIELSEN, J. ; TURKEZ, H. ; SHOAIE, S. ; UHLEN, M. et al.** A Gene Co-Expression Network-Based Drug Repositioning Approach Identifies Candidates for Treatment of Hepatocellular Carcinoma. *Cancers (Basel).,* 2022, vol. 14, 1573
- **ZHOU, Y. ; HOU, Y. ; SHEN, J. ; HUANG, Y. ; MARTIN, W. ; CHENG, F.** Network-based drug repurposing for novel coronavirus 2019-nCoV/SARS-CoV-2. *Cell Discov.,* 2020, vol. 6, 14